# EUROPÄISCHE PATENTANMELDUNG

(11) **EP 4 118 963 A1**
(43) Veröffentlichungstag der Anmeldung: **18.01.2023**
(21) Anmeldenummer: 21185611.7
(22) Anmeldetag: 14.07.2021
(51) Int. Cl.: A01K 67/033

(54) **ANORDNUNG ZUR ZUCHT VON INSEKTEN UND VERFAHREN ZUR ZUCHT VON INSEKTEN UNTER VERWENDUNG DER ANORDNUNG**

(71) Anmelder: INOVA Protein GmbH, 18059 Rostock (DE)
(72) Erfinder: Funck, Nico, 18055 Rostock (DE); Schiemann, Raijana, 18057 Rostock (DE); Baudisch, Christian, 18059 Rostock (DE)
(74) Vertreter: Grünbaum, Annekathrin

(57) **Zusammenfassung**

Die Erfindung betrifft eine Anordnung zur Zucht von Insekten und ein Verfahren zur Zucht von Insekten unter Verwendung der Anordnung.

Es ist daher Aufgabe der Erfindung die Nachteile des Standes der Technik zu beseitigen und eine Anordnung zur Zucht von Insekten bereitzustellen, mittels welcher eine Fütterung und/oder Kontrolle automatisiert erfolgen kann. Es ist ferner Aufgabe der Erfindung ein Verfahren bereitzustellen, welches automatisiert und vereinfacht eine Zucht von Insekten ermöglicht.

Die Lösung dieser Aufgabe erfolgt durch die in den Ansprüchen aufgeführten Merkmale.

## Beschreibung

Die Erfindung betrifft eine Anordnung zur Zucht von Insekten und ein Verfahren zur Zucht von Insekten unter Verwendung der Anordnung.

Die Erfindung befasst sich mit einer vollautomatisierte Insekten-Zucht und deren Verarbeitung. Insbesondere umfasst die Erfindung die vollautomatisierte Zucht und Verarbeitung von Insekten zu Insektenmehl, eine spezifische, wissenschaftliche Zuchtstrategie, Hygienekonzepte, sowie die Verwendung einzigartiger Substrate. Die Verwendung von Insektenmehl in der Ernährung von Mensch und Tier gewinnt zunehmend an Bedeutung.

Der Markt für Insektenmehlhersteller unterteilt sich in zwei Verwendungsbereiche. Zunächst gibt es lizensierte Betriebe, die ihre Insekten für die Ernährung von Menschen vertreiben dürfen.

Des Weiteren gibt es Insektenfarmen, welche sich auf den Tierfutterbereich und hier besonders auf Petfood, Geflügelhaltung und Aquakulturen spezialisiert haben.

Fundamental unterschiedlich ist die Aufzucht und die Verarbeitung von verschiedenen Insektenarten, daher müssen Systeme direkt an die spezielle Insektenart angepasst werden und können nur teilweise oder kaum auf andere Arten transferiert werden.

Außerdem gibt es gravierende Unterschiede in der Aufzucht und Verarbeitung für die Produktion von Lebensmitteln im Vergleich zum Tierfutter. Angefangen von der Fütterung, bis hin zur Verarbeitung müssen hier andere Standards und Qualitätsmerkmale angewandt werden.

Der Betrieb von Insektenaufzuchten wird oft rein händisch betrieben, wodurch der Personalaufwand enorm ist. Vor allem die Fütterung ist eines der wichtigsten Kernelemente in der Aufzucht von Insekten.

Im Stand der Technik ist auch bekannt, dass wärmeabgebene Lampen verwendet werden können, um eine optimalen Temperatur in den einzelnen Zuchtboxen zu erzielen.

Die Druckschrift EP2986107A1 offenbart ein Verfahren und ein System zum Züchten von Insekten unter Verwendung einer Vielzahl einzelner Kisten, wobei mindestens ein Teil jeder Kiste mit einem Substrat gefüllt ist, das Futtermittel und unreife Phasen von Insekten enthält. Ebenfalls vorgesehen ist ein Klimabereich, in dem die Kisten mit einem Belüftungssystem untergebracht sind. In dem System ist ein Fördersystem enthalten, mit dem die Kisten aus dem Klimabereich entnommen und dorthin zurückgeführt werden können. Ein Beobachtungssystem zum Erhalten von Beobachtungen, einschließlich Daten und Messungen, und stromabwärts davon ist eine Rohstoffversorgungsstation entlang des Fördersystems angeordnet. In dieser Offenbarung werden Kistenstapel verwendet, welche zunächst abgestapelt werden müssen und jede einzelne Kiste wird gefüttert und überprüft, ob diese geerntet werden muss. Das hat zur Folge, dass ein großer Arbeitsaufwand in dem Auf- und Abstapeln der Kisten benötigt wird und der manuelle Verarbeitungsbereich ständig hochgefahren werden muss.

Die Offenbarung der Patentschrift DE102019121102B3 betrifft eine Vorrichtung und ein Verfahren zur Insektenzucht, wobei sich die Vorrichtung dadurch auszeichnet, dass die Vorrichtung wenigstens einen Stapel von Kisten, welcher sich auf einem Chassis befindet, und ein Portal umfasst, wobei das Portal wenigstens über ein Versorgungsmodul, ein Reinigungsmodul, einen Vertikaltransporter nach oben, einen Vertikaltransporter nach unten, einen Quertransporter oben, sowie einen Quertransporter unten verfügt, so dass mittels des Portals ein zyklisches Umschichten und eine Versorgung und Reinigung der Insekten innerhalb der Kisten, innerhalb des wenigstens einen Stapels, ermöglicht ist.

Nachteilig im Stand der Technik ist, dass aufgestapelte Zuchtboxen für jede Fütterung und Kontrolle abgestapelt werden müssen. Dabei handelt es sich um einen zeitaufwendigen Prozess.

Im Stand der Technik ist keine Lösung offenbart, welche es ermöglicht bei der Fütterung und Kontrolle der Insekten-Zucht eine Abstapelung zu vermeiden.

### Darstellung der Erfindung

Es ist daher Aufgabe der Erfindung die Nachteile des Standes der Technik zu beseitigen und eine Anordnung zur Zucht von Insekten bereitzustellen, mittels welcher eine Fütterung und/oder Kontrolle automatisiert erfolgen kann. Es ist ferner Aufgabe der Erfindung ein Verfahren bereitzustellen, welches automatisiert und vereinfacht eine Zucht von Insekten ermöglicht.

Es ist auch Aufgabe eine Anordnung bereit zu stellen mittels welcher eine vereinfachte automatisierte Fütterung und Kontrolle erfolgt. Ferner soll ein aufwendiges Umschichten der Kisten in den Stapeln verhindert werden, um ein Zeit- und Kostenvorteil zu erzielen und um die Zuchtkisten keinen weiteren unnötigen Erschütterungen auszusetzen.

Die Lösung dieser Aufgabe erfolgt durch die in den Ansprüchen aufgeführten Merkmale.

Gemäß verschiedenen Ausführungsformen weist die erfindungsgemäße Anordnung zur Zucht von Insekten zumindest einen Zuchtboxenstapel auf, welcher an einem Lagerstandort gelagert wird. Der Zuchtboxenstapel weist mindestens zwei Zuchtboxen auf. In den Zuchtboxen werden die Insekten, bevorzugt Mehlwürmer, gehalten und aufgezogen und gezüchtet. Die Zuchtboxen sind übereinander in vertikaler Richtung in einem Zuchtboxenstapel gestapelt, derart dass ein Zwischenraum (erster Zwischenraum) zwischen den mindestens zwei Zuchtboxen gebildet wird. Ferner weist die Anordnung gemäß verschiedenen Ausführungsformen zumindest eine Station zur Fütterung und/oder Kontrolle der Insekten in den Zuchtboxen auf. Ferner weist die Anordnung zumindest ein Transportsystem für einen Transport des zumindest einen Zuchtboxenstapels vom Lagerstandort zur Station auf. Die Station weist ferner zumindest eine Fütterungsvorrichtung zur Fütterung der einzelnen Zuchtboxen und/oder eine Vorrichtung zur Kontrolle der einzelnen Zuchtboxen auf. Die Fütterungsvorrichtung weist einen Rührkessel und ein Rohrsystem auf, wobei das Rohrsystem zumindest ein Rohr aufweist. Der Rührkessel der Fütterungsvorrichtung weist zumindest einen Auslass aufweist, wobei der zumindest eine Auslass mit einem ersten Rohrende des zumindest eines Rohres oder Rohrsystems verbunden ist. Ein zweites Rohrende des zumindest einen Rohres oder des Rohrsystems ragt in den ersten Zwischenraum des Zuchtboxenstapels hinein.

Gemäß verschiedenen Ausführungsformen weist die Fütterungsvorrichtung einen Bewegungsmechanismus auf, wobei der Bewegungsmechanismus derart ausgestaltet ist, dass das zumindest eine Rohr des Rohrsystems und/oder das gesamte Rohrsystem eine vertikale Bewegung erfährt, so dass das zweite Rohrende von dem ersten Zwischenraum in einen zweiten Zwischenraum bewegt wird.

Gemäß verschiedenen Ausführungsformen weist der Bewegungsmechanismus zumindest einen Schlitten und eine Führung, beispielsweise Führungsschienen, auf, wobei der Schlitten entlang der Führung in vertikaler Richtung beweglich gelagert ist. Die Führung ist parallel zum Zuchtboxenstapel gelagert, so dass der Schlitten entlang der Zuchtboxen in einem Zuchtboxenstapel bewegt werden kann. Die Bewegung kann manuell oder mittels einer Antriebsvorrichtung automatisiert erfolgen.

Gemäß verschiedenen Ausführungsformen weist das Rohrsystem zumindest eine Pumpeinrichtung auf, wobei das Pumpsystem für einen Betrieb von zumindest viskosen Flüssigkeiten ausgelegt ist. Hierdurch kann das Futtermittel besser vom Rührkessel bis hin zu den Zuchtkisten transportiert werden. Es ist auch denkbar über das Pumpsystem Wasser durch das Rohrsystem zu leiten, um eine Reinigung des Rohrsystems und/oder des Rührkessels durchführen.

Gemäß verschiedenen Aufführungsformen ist am zweiten Rohrende ein Ventil angeordnet. Hierdurch kann eine bessere und genauere Futtermittelabgabe erreicht werden.

Gemäß verschiedenen Ausführungsformen ist am zweiten Rohrende ein Ausfahrmechanismus angeordnet. Mittels des Ausfahrmechanismus kann eine Verlängerung des zweiten Rohrendes erfolgen, so dass das zweite Rohrende in einen Zwischenraum hineingeführt werden kann.

Gemäß verschiedenen Ausführungsformen kann die Station zumindest eine Aufnehmung zur Aufnahme des zumindest einen Zuchtboxenstapels aufweisen. Hierdurch kann ein besserer Halt und/oder eine bessere Platzierung des Zuchtboxenstapels erfolgen.

Bevorzugt weist das Rohrsystem mehrere Rohre und/oder mehrere zweite Rohrenden auf, so dass mehrere Zwischenräume/Zuchtboxen gleichzeitig befüttert werden können.

Gemäß verschiedenen Ausführungsformen weist/weisen die Anordnung zur Zucht von Insekten und/oder die Vorrichtung zur Kontrolle und/oder die Fütterungsvorrichtung eine Sensoreinheit mit zumindest einem Sensor und/oder eine Auswerteinheit und/oder eine Speichereinheit und/oder eine Ausgabeeinheit.

Der zumindest eine Sensor ist eine Kamera oder ein Sensor zur Gewichtsaufnahme oder ein Feuchtigkeitssensor oder ein Temperatursensor. Die Verwendung weiterer Sensoren oder einer Kombination mehrerer Sensoren ist denkbar. Die Vorrichtung zur Kontrolle und/oder die Sensoreinheit und/oder der zumindest eine Sensor der Sensoreinheit ist/sind am zweiten Rohrende angeordnet. Hierdurch können direkt in den Zuchtkisten und/oder im Zwischenraum zwischen den Zuchtkisten Messwerte und/oder Daten aufgenommen werden. Es ist auch denkbar Sensoren der Sensoreinheit in, an und/oder unter der Aufnehmung der Station anzuordnen. Weitere Orte zur Platzierung der Sensoreinheit in der Anordnung sind denkbar.

Die Aufgabe wird auch gelöst mittels eines Verfahrens zur Zucht von Insekten unter Verwendung einer Anordnung zur Zucht von Insekten gemäß Anspruch 1.

Das erfindungsgemäße Verfahren weist folgende Verfahrensschritte auf:
a) Transport eines Zuchtboxenstapels mittels des Transportsystems vom Lagerstandort zur Station,
b) Füttern der Insekten in den einzelnen Zuchtboxen mittels der Fütterungsvorrichtung und/oder
c) Aufnahme von Messwerten und/oder Daten zur Qualitätskontrolle und Auswerten der Messwerte und/oder Daten mittels der Vorrichtung zur Kontrolle,
e) Rücktransport des Zuchtboxenstapels mittels des Transportsystems an den Lagerstandort oder Transport des Zuchtboxenstapels an eine zweite Station zur weiteren Kontrolle.

Gemäß verschiedener Ausführungsformen werden für den Transport Roboter verwendet, welche den innerbetrieblichen Transport der Zuchtboxen und/oder Arbeitsschritte des Verfahrens übernehmen. Die Verwendung weiterer Transportmittel ist denkbar, beispielsweise Rollbänder, Greifroboter, welche an der Decke entlang von Schienen sich fortbewegen oder ähnliches.

Gemäß verschiedener Ausführungsformen werden für die Qualitätskontrolle Daten und/oder Messwerte, beispielsweise in Bezug auf Temperatur, Gewicht, Feuchtigkeit und/oder Gesundheitszustand aufgenommen. Hierzu kann eine Sensoreinheit der Anordnung und/oder der Vorrichtung zur Kontrolle und/oder der Fütterungsvorrichtung verwendet werden.

Gemäß verschiedenen Ausführungsformen wird für die Ermittlung des Gesundheitszustandes ein photogrammetrisches Verfahren angewendet. Für die Durchführung des photogrammetrische Verfahren wird bevorzugt eine Kamera verwendet, welche Schwarz/Weiß-Kontraste in den Zuchtboxen erkennt.

Gemäß verschiedenen Ausführungsformen erfolgt eine zusätzlich Einkreuzung eines neues Insektenstamm mindestens alle sechs Monate. Andere Zeitintervalle sind denkbar

Gemäß verschiedenen Ausführungsformen erfolgt eine Auswertung der Messwerte und/oder Daten. Ferner kann basierend auf der Auswertung der Messwerte und/oder Daten eine Ermittlung einer Fütterungsmenge, eine Ermittlung einer nächsten Fütterungszeit und/oder eine Ermittlung einer nächsten Kontrolle erfolgen.

Vorteilhaft an der erfindungsgemäßen Anordnung und dem erfindungsgemäßen Verfahren ist, das große Totalausfälle, aufgrund der genetischen Einengung des Genpools, ausbleiben und die Wachstumsgeschwindigkeit der Larven gesteigert wird. Um den Effekt der Heterosis zu nutzen und um eine Einengung des Genpools zu vermeiden, wird zusätzlich alle 6 Monate ein neuer Stamm mit eingekreuzt.

Die Zuchtboxen sind extra für die Insektenzucht angefertigt. Dabei sind diese Zuchtboxen insbesondere für die Zucht des Gelben Mehlwurms geeignet. Über gesonderte Öffnungen verfügen die Zuchtboxen die Eigenschaft keine hohe Feuchtigkeit bilden zu lassen. Gerade dies ist ein sehr wichtiger Vorteil für die Zucht, weil jegliche Feuchte sehr schädlich ist und zur Entstehung von Krankheiten führt. Daneben ist es mit den entwickelten Zuchtboxen mögliche sie vertikal zu stapelt. Sie haben an der Seitenkante Stelzen worauf sich die nächste Zuchtbox raufstellen lässt. Durch diese Stelzen ist immer noch eine sehr gute Luftzirkulation möglich, wodurch immer genügen Sauerstoff für die Insekten vorhanden ist.

Durch die Automatisierung wird der innerbetriebliche Transport im geschlossenen System bevorzugt von Robotern, insbesondere von Breeding-Robotern, übernommen, die sich autonom in der Produktion bewegen und alle Arbeitsschritte erledigen können. Die Arbeitswege der Roboter werden beispielsweise mittels Magnetstreifen statisch festgelegt. Die Arbeitspakete werden von der Steuereinheit direkt an die Roboter gesendet, welche dann autonom von den Robotern ausgeführt werden. Die Roboter sind so programmiert, dass sie alle Produktionsprozesse präzise ausführen. Durch die spezielle erfindungsgemäße Anordnung können sich wiederholenden Aufgaben und Arbeiten automatisch abgearbeitet werden. Handarbeite ist nicht mehr nötig. Nur bei Stichprobenkontrolle und/oder in einer zweiten Station, in welche die aussortierten Zuchtboxen befördert werden, wenn die Auswertung der Messwerte und Daten eine Nachkontrolle erfordern, kann ein manueller Eingriff erforderlich sein.

Die Station ist eine speziell konzipierte Bearbeitungsstation, die alle erforderlichen Arbeitsschritte automatisiert und effizient erledigt. Hauptaufgabe dieser Station ist die Fütterung der einzelnen Zuchtboxen nach ihren spezifischen Anforderungen. Denn abhängig von dem Lebensstadium benötigen die Insekten eine spezifische Zusammensetzung der Futtermittel, die automatisch an der Station erfolgt. Daneben kann auch das Gewicht der einzelnen Zuchtboxen ermittelt werden, um auf die Wachstumsrate schließen zu können. Diese werden für die statistische Auswertungen genutzt, woraus sich die optimale Fütterungsmenge, sowie der Zeitpunkt der Verarbeitung ergibt. Ebenso wird die Feuchtigkeit innerhalb der Zuchtboxen erfasst. Sie muss immer konstant niedrig gehalten werden, um Fäulnis zu verhindern. Nachdem die Bearbeitung an der Station abgeschlossen wurde, wird der Batch wieder an den Lagerstandort gefahren und alle kennzeichnenden Daten aktualisiert.

Anhand eins photogrammetrischen Verfahrens werden die Vitalwerte der einzelnen Zuchtboxen an der Station erfasst, um mögliche Krankheitsherde frühzeitig zu erkennen. Dies geschieht mit einer speziellen Kamera, die Schwarz/Weiß-Kontraste in den Zuchtboxen erkennen kann. Durch das spezifische Lichtspektrum erkennt man alle Verfärbungen der Insekten. Tote Larven färben sich komplett schwarz und Krankheiten äußern sich ebenfalls in einer Verfärbung der Larven. Dadurch werden sie für Kamera-Sensorik sehr gut erkennbar. Sobald eine Zuchtbox den Grenzwert erreicht hat, wird sie von dem zugehörigen Zuchtboxenstapel getrennt und weiter untersucht

### Ausführung der Erfindung

Die Erfindung wird anhand eines/mehrerer Ausführungsbeispiels näher erläutert. Hierzu zeigen
- Figur 1: Teil einer Anordnung zur Zucht von Insekten
- Figur 2: Detailansicht aus Figur 1

In der Beschreibung wird auf die beigefügten Zeichnungen Bezug genommen, in denen zur Veranschaulichung spezifische Ausführungsformen gezeigt sind, in denen die erfindungsgemäße Anordnung ausgeübt werden kann. In dieser Hinsicht wird eine Richtungsterminologie wie etwa "oben", "unten" usw. mit Bezug auf die Orientierung der beschriebenen Zeichnungen verwendet. Die Richtungsterminologie dient der Veranschaulichung und ist auf keinerlei Weise einschränkend.

Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In den Figuren werden identische oder ähnliche Elemente mit identischen Bezugszeichen versehen, soweit dies zweckmäßig ist.

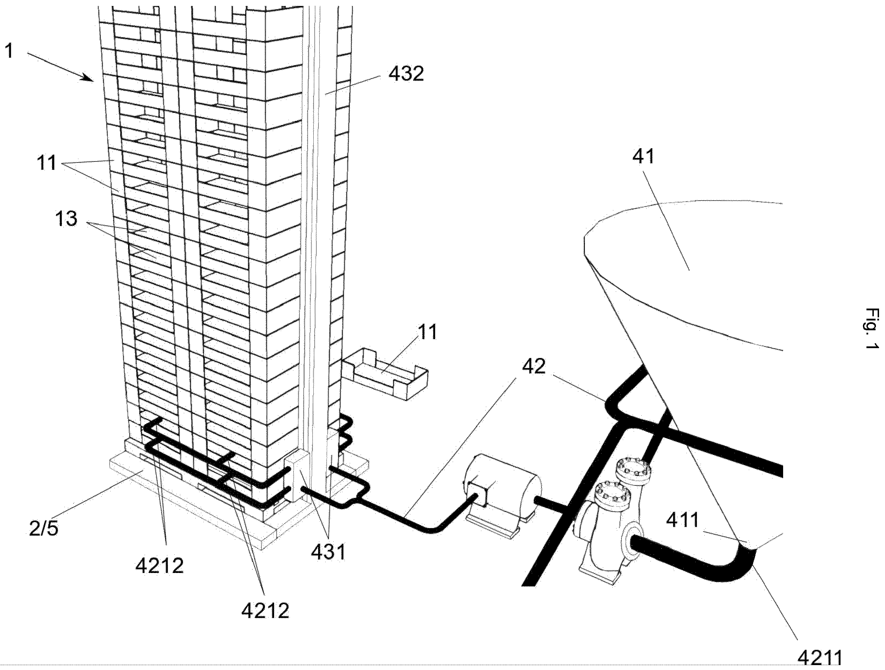

In Figur 1 ist eine erfindungsgemäße Anordnung zur Zucht von Insekten dargestellt. Die Anordnung weist gemäß verschiedenen Ausführungsformen zumindest einen Zuchtboxenstapel 1 an einem Lagerstandort 31 (hier nicht dargestellt) auf, wobei der Zuchtboxenstapel 1 mindestens zwei Zuchtboxen 11 aufweist, welche übereinander in vertikaler Richtung gestapelt sind, derart dass ein erster Zwischenraum 13 zwischen den mindestens zwei Zuchtboxen 11 gebildet wird. Ferner weist die Anordnung zumindest eine Station 2 zur Fütterung und/oder Kontrolle auf. Mittels eines Transportsystems 3 (hier nicht dargestellt) erfolgt ein Transport des zumindest einen Zuchtboxenstapels 1 vom Lagerstandort 31 zur Station 2. Die zumindest eine Station 2 weist zumindest eine Fütterungsvorrichtung 4 zur Fütterung der einzelnen Zuchtboxen 11 und/oder eine Vorrichtung 6 (hier nicht dargestellt) zur Kontrolle der einzelnen Zuchtboxen 11 auf. Die Fütterungsvorrichtung 4 weist, wie in Figur 1 dargestellt, einen Rührkessel 41 auf. Der Rührkessel 41 ist mit einem Rohrsystem 42 mit zumindest einem Rohr 421 verbunden. Der Rührkessel 41 weist zumindest einen Auslass 411 auf, wobei der zumindest eine Auslass 411, wie in Figur 1 dargestellt mit dem Rohrsystem 421 verbunden ist, wobei das erste Rohrende 4211 des zumindest eines Rohres 421 oder des Rohrsystems 42 mit dem Auslass 411 des Rührkessels 41 verbunden ist, und wobei ein zweites Rohrende 4212 des zumindest einen Rohres 421 oder des Rohrsystems 42 in den ersten Zwischenraum 13 hineinragt.

Gemäß verschiedenen Ausführungsformen weist die Fütterungsvorrichtung 4 einen Bewegungsmechanismus 43 auf, wobei der Bewegungsmechanismus 43 derart ausgestaltet ist, dass das zumindest eine Rohr 421 des Rohrsystems 42 und/oder das Rohrsystem 42 eine vertikale Bewegung erfährt, so dass das zweite Rohrende 4212 des zumindest einen Rohres 421 oder des Rohrsystems 42 von dem ersten Zwischenraum 13 in einen zweiten Zwischenraum 131 bewegt wird.

Gemäß verschiedenen Ausführungsformen weist der Bewegungsmechanismus 43 zumindest einen Schlitten 431 und eine Führung 432 auf, wobei die Führung parallel zum Zuchtboxenstapel 1 angeordnet ist, wobei der Schlitten 431 entlang der Führung 432 in vertikaler Richtung beweglich gelagert ist.

Gemäß verschiedenen Ausführungsformen weist das Rohrsystem 42 zumindest eine Pumpeinrichtung 423 auf.

Gemäß verschiedenen Ausführungsformen ist am zweiten Rohrende 4212 ein Ventil angeordnet.

Gemäß verschiedenen Ausführungsformen ist am zweiten Rohrende 4212 ein Ausfahrmechanismus angeordnet mittels welchem eine Verlängerung des zweiten Rohrendes 4212 erfolgt, so dass das zweite Rohrende 4212 in einen Zwischenraum 13/131 hineingeführt werden kann.

Gemäß verschiedenen Ausführungsformen weist die Station 2 zumindest eine Aufnehmung 5 zur Aufnahme des zumindest einen Zuchtboxenstapels 1 auf.

Gemäß verschiedenen Ausführungsformen weist/weisen die Anordnung zur Zucht von Insekten und/oder die Vorrichtung zur Kontrolle 6 eine Sensoreinheit und/oder eine Auswerteinheit und/oder eine Speichereinheit und/oder eine Ausgabeeinheit auf, wobei die Sensoreinheit bevorzugt eine Kamera ausweist.

Die Rohre 421 des Rohrsystems 42 sind gemäß verschiedenen Ausführungsformen flexibel ausgestaltet. Flexible Schläuche werden auch als Rohr definiert.

Die ersten Rohrenden 4211 sind bevorzugt mit einem Ausfahrmechanismus ausgestattet, der ermöglichen soll, dass das Ende der Schläuche (das erste Rohrende 4211) sich in die Zuchtboxen 11 bzw. in die Zwischenräume 13 und wieder herausbewegen kann, ohne dabei mit einer der Zuchtboxen 11 in Berührung zu kommen. Für eine Dosierung des Futtermittels werden Ventile am ersten Rohrende angebracht, die sich ab einer bestimmten Menge schließen können. So kann sichergestellt werden, dass die Mehlwürmer die perfekte Menge an Futtermittel erhalten.

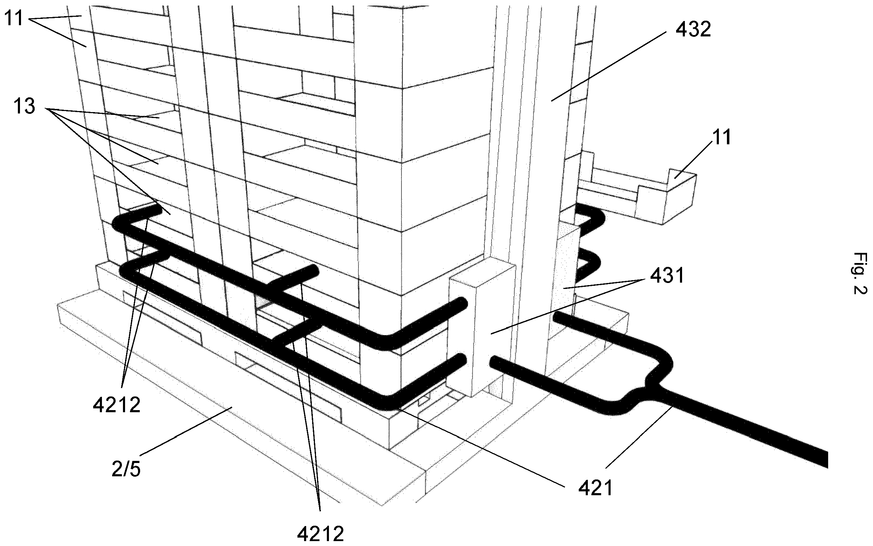

Figur 2 zeigt einen Ausschnitt der Figur 1 etwas detaillierter. Es ist ein Zuchtboxenstapel 1 in der Station 2 dargestellt. Zur Verfügung steht ein Rührkessel 41 (in Figur 2 nicht dargestellt), der das benötigte Futtermittel im richtigen Verhältnis vermischt. Damit das Futtermittel zu den Mehlwürmern transportiert werden kann, steht eine Pumpe, beispielsweise eine Exzenterschneckenpumpe (Pumpe für viskose Produkte) zur Verfügung. Der Rührkessel 41 benötigt im besten Falle am unteren Ende einen Anschluss für die Pumpe, sodass diese auch bei einem niedrigen Futterstand im Rührkessel genügend Futter ansaugt, ohne dabei Luft zusätzlich einzusaugen, welche die Fütterung manipulieren würde. Das Futter wird über die Pumpe über das Rohrsystem 42 hin zu den Zuchtboxen 11 transportiert. Die Station weist eine Aufnehmung 5 auf, in die zumindest ein Zuchtboxenstapel 1 abgestellt werden kann, wie hier dargestellt ist. Die Aufnehmung 5 kann beispielsweise derart ausgestaltet sein, dass eine handelsübliche Europalette abgestellt werden kann. Auf der Palette befinden sich die gestapelte Zuchtboxen 11, in denen sich die Mehlwürmer befinden. Dabei kann ein Zuchtboxenstapel 1 insgesamt eine Höhe von bis zu drei Metern erreichen. Die Fütterungsvorrichtung 4 weist gemäß verschiedenen Ausführungsformen eine Führung 432 auf, die bevorzugt auch zur Stabilisierung des Bewegungsmechanismus geeignet ist, beispielsweise eine Säule. Die Führung 432 ist beispielsweise mittig am vorderen Teil des Zuchtboxenstapels 1 gelagert, wenn dieser in der Aufnehmung 5 angeordnet ist. An jeweils zwei Seiten der Säule befinden sich Schienen 432, die eine Bewegung in vertikaler Richtung ermöglichen. An den Schienen 432 ist eine Apparatur, beispielsweise ein Schlitten 431, angebracht, die die vertikale Bewegung automatisch durchführen soll. Von dieser Apparatur führen Schläuche weg. Durch diese Schläuche soll das von der Pumpe transportierte Futtermittel gelangen. Die Schläuche führen, wie in Figur 1 zu sehen ist, in die Zuchtboxen 13. Die Schläuche sollen dabei mit einem Ausfahrmechanismus ausgestattet sein, der ermöglichen soll, dass das Ende der Schläuche sich in die Zuchtboxen 11 und wieder herausbewegen kann, ohne dabei mit einer Zuchtboxen 11 in Berührung zu kommen. Für eine Dosierung des Futtermittels werden bevorzugt Ventile am Ende der Schläuche angebracht, die sich ab einer bestimmten Menge schießen. So kann sichergestellt werden, dass die Mehlwürmer die perfekte Menge an Futtermittel erhalten.

Beispielsweise per Knopfdruck oder automatisch mittels eines Sensors, der erkennt, dass ein neuer Zuchtboxenstapel 1 in der Station 2 abgestellt wurde, wird die Fütterungsvorrichtung 4/Station 2/Vorrichtung 6 zur Kontrolle "eingeschaltet". Das Futter wird im Rührkessel 41 vermischt und/oder angerührt. Über das Rohrsystem 42 wird das Futter in die erste/die ersten Zuchtboxen 11 (beispielsweise die unteren, wie in Figur 1 dargestellt) eingeführt, beispielsweise eingesprüht. Nach der Fütterung fährt der Schlitten 431 eine vordefinierte Distanz in die Vertikale, damit die nächsten Boxen bedient werden können. Dieser Mechanismus wird solange wiederholt, bis alle Zuchtboxen 11 mit Futtermittel bedient wurden. Der Bewegungsmechanismus 4 der Fütterungsvorrichtung 4 fährt in die Ausgangsposition zurück und erzeugt ein Signal (optisch und/oder akustisch) mittels eine Ausgabeeinheit, welches die Beendigung des Prozesses vermitteln soll. Für die erneute Benutzung der Fütterungsvorrichtung 4 und/oder der Station 2 muss die vorhandene Palette mit Boxen (der Zuchtboxenstapel 1) entfernt werden und durch eine Palette mit nicht gefütterten Boxen (einem weiteren Zuchtboxenstapel 1) ersetzt werden.

Ein wichtiger Parameter bei dieser automatisierten Variante ist die Sicherstellung, dass immer ausreichend Futtermittel aus dem Rührkessel 41 gepumpt werden kann. Das heißt, dass für den Füllstand des Rührkessels 41 eine Kontrolle implementiert ist.

Für die Reinigung kann des Weiteren eine einfache Pumpe verwendet werden, die Wasser durch die Schläuche pumpt.

In der Station 2 erfolgt zusätzlich zur Fütterung oder alternativ eine Qualitätskontrolle der Insekten in den Zuchtboxen 11. Bei den Insekten, beispielsweise bei Mehlwürmern, kann es vereinzelt zu Krankheiten kommen, die auch zum Tode führen können. Sollten Mehlwürmer sterben, verändert sich die Farbe des Körpers von einem recht hellen Gelb zu einem fast schon schwarzen Körper. Schon während des Krankheitsverlaufes verfärbt sich der Körper des Mehlwurms dahingehend, dass er etwas dunkler wird. Um die Entstehung von Krankheiten vorzeitig zu erkennen und Gegenmaßnahmen einleiten zu können, sind Sensoren einer Sensoreinheit an der Vorrichtung 6 zur Kontrolle angeordnet. Die Sensoreinheit und/oder die Vorrichtung zur Kontrolle 6 können an der Fütterungsvorrichtung und/oder an der Station angeordnet sein. Der Ort der Befestigung könnten beispielsweise die zweiten Rohrenden 4212 sein, die jede Zuchtbox 11 mindestens einmal abfahren. Als Sensoren kann eine Kamera verwendet werden, welche für ein bestimmtes Spektrum konzipiert ist. Das bedeutet, dass diese dann auch leichte farbliche Unterschiede bemerken kann.

### Bezugszeichen

- 1: Zuchtboxenstapel
11 Zuchtbox
13 Zwischenraum

- 2: Station
- 3: Transportsystem
31 Lagerstandort

- 4: Fütterungsvorrichtung
- 41: Rührkessel
411 Auslass
42 Rohrsystem
421 Rohr
4211 erstes Rohrende
4212 zweites Rohrende
43 Bewegungsmechanismus
431 Schlitten
432 Führung
5 Aufnehmung
6 Vorrichtung zur Kontrolle

## Patentansprüche

1. Anordnung zur Zucht von Insekten aufweisend
zumindest ein Zuchtboxenstapel (1) an einem Lagerstandort (31), wobei dieser mindestens zwei Zuchtboxen (11) aufweist, welche übereinander in vertikaler Richtung gestapelt sind, derart dass ein erster Zwischenraum (13) zwischen den mindestens zwei Zuchtboxen (11) gebildet wird,
zumindest eine Station (2) zur Fütterung und/oder Kontrolle,
zumindest ein Transportsystem (3) für einen Transport des zumindest einen Zuchtboxenstapels (1) vom Lagerstandort (31) zur Station (2),
wobei die zumindest eine Station (2) zumindest eine Fütterungsvorrichtung (4) zur Fütterung der einzelnen Zuchtboxen (11) aufweist und/oder eine Vorrichtung (6) zur Kontrolle der einzelnen Zuchtboxen (11),
wobei die Fütterungsvorrichtung (4) einen Rührkessel (41) und ein Rohrsystem (42) mit zumindest einem Rohr (421) aufweist,
wobei der Rührkessel (41) zumindest einen Auslass (411) aufweist, wobei der zumindest eine Auslass (411) mit einem ersten Rohrende (4211) des zumindest eines Rohres (421) oder des Rohrsystems (42) verbunden ist, und wobei ein zweites Rohrende (4212) des zumindest einen Rohres (421) oder des Rohrsystems (42) in den ersten Zwischenraum (13) hineinragt.

2. Anordnung zur Zucht von Insekten gemäß Anspruch 1, **dadurch gekennzeichnet, dass**
die Fütterungsvorrichtung (4) einen Bewegungsmechanismus (43) aufweist, wobei der Bewegungsmechanismus (43) derart ausgestaltet ist, dass das zumindest eine Rohr (421) des Rohrsystems (42) und/oder das Rohrsystem (42) eine vertikale Bewegung erfährt, so dass das zweite Rohrende (4212) des zumindest einen Rohres (421) oder des Rohrsystems (42) von dem ersten Zwischenraum (13) in einen zweiten Zwischenraum (131) bewegt wird.

3. Anordnung zur Zucht von Insekten gemäß Anspruch 1, **dadurch gekennzeichnet, dass**
der Bewegungsmechanismus (43) zumindest einen Schlitten (431) und eine Führung (432) aufweist, wobei die Führung (432) parallel zum Zuchtboxenstapel (1) angeordnet ist, wobei der Schlitten (431) entlang der Führung (432) in vertikaler Richtung beweglich gelagert ist.

4. Anordnung zur Zucht von Insekten gemäß Anspruch 1, **dadurch gekennzeichnet, dass**
das Rohrsystem (42) zumindest eine Pumpeinrichtung (423) aufweist.

5. Anordnung zur Zucht von Insekten gemäß Anspruch 1, **dadurch gekennzeichnet, dass**
am zweiten Rohrende (4212) ein Ventil (44) angeordnet ist.

6. Anordnung zur Zucht von Insekten gemäß Anspruch 1, **dadurch gekennzeichnet, dass**
am zweiten Rohrende (4212) ein Ausfahrmechanismus (45) angeordnet ist mittels welchem eine Verlängerung des zweiten Rohrendes (4212) erfolgt, so dass das zweite Rohrende (4212) in einen Zwischenraum (13/131) hineingeführt werden kann.

7. Anordnung zur Zucht von Insekten gemäß Anspruch 1, **dadurch gekennzeichnet, dass**
die Station (2) zumindest eine Aufnehmung (5) zur Aufnahme des zumindest einen Zuchtboxenstapels (1) aufweist.

8. Anordnung zur Zucht von Insekten gemäß Anspruch 1, **dadurch gekennzeichnet, dass**
die Anordnung zur Zucht von Insekten und/oder die Vorrichtung zur Kontrolle (6) eine Sensoreinheit und/oder eine Auswerteinheit und/oder eine Speichereinheit und/oder eine Ausgabeeinheit aufweist/aufweisen.

9. Anordnung zur Zucht von Insekten gemäß Anspruch 8, **dadurch gekennzeichnet, dass**
die Sensoreinheit eine Kamera ausweist.

10. Verfahren zur Zucht von Insekten unter Verwendung einer Anordnung zur Zucht von Insekten gemäß Anspruch 1, Verfahren aufweisend folgende Verfahrensschritte:
a) Transport eines Zuchtboxenstapels (1) mittels des Transportsystems (3) vom Lagerstandort (31) zur Station (2),
b) Füttern mittels der Fütterungsvorrichtung (4) der Insekten in den einzelnen Zuchtboxen (11) und/oder
c) Aufnahme von Messwerten und/oder Daten zur Qualitätskontrolle und Auswerten der Messwerte und/oder Daten mittels der Vorrichtung zur Kontrolle (6),
e) Rücktransport des Zuchtboxenstapels (1) mittels des Transportsystems (3) an den Lagerstandort (31) oder Transport des Zuchtboxenstapels (1) an eine zweite Station (21) zur weiteren Kontrolle.

11. Verfahren gemäß Anspruch 10, **dadurch gekennzeichnet, dass** für einen Transport Roboter verwendet werden, welche den innerbetrieblichen Transport von Zuchtboxen übernehmen und/oder Arbeitsschritte des Verfahrens übernehmen.

12. Verfahren gemäß Anspruch 10, **dadurch gekennzeichnet, dass** für die Qualitätskontrolle Daten und/oder Messwerte in Bezug auf Gewicht, Feuchtigkeit und/oder Gesundheitszustand aufgenommen werden.

13. Verfahren gemäß Anspruch 12, **dadurch gekennzeichnet, dass** für die Ermittlung des Gesundheitszustandes ein photogrammetrisches Verfahren angewendet wird.

14. Verfahren gemäß Anspruch 13, **dadurch gekennzeichnet, dass** für das photogrammetrische Verfahren eine Kamera verwendet, welche Schwarz/Weiß-Kontraste in den Zuchtboxen (11) erkennt.

15. Verfahren gemäß einem der Ansprüche 10-15 **dadurch gekennzeichnet, dass** eine Auswertung der Messwerte und/oder Daten erfolgt und/oder dass basierend auf der Auswertung der Messwerte und/oder Daten eine Ermittlung einer Fütterungsmenge, eine Ermittlung einer nächsten Fütterungszeit und/oder eine Ermittlung einer nächsten Kontrolle erfolgt.
